# EUROPEAN PATENT APPLICATION

(11) **EP 1 610 485 A2**
(43) Date of publication of application: **28.12.2005**
(21) Application number: 05105580.4
(22) Date of filing: 23.06.2005
(51) Int. Cl.: H04L 1/00

(54) **Device, system and method for error detection of IN-VIVO data**

(30) Priority: 23.06.2004 US 873497
(71) Applicant: Given Imaging Ltd., Yoqneam 20692 (IL)
(72) Inventor: BETTESH, Ido, 34788, Haifa (IL)
(74) Representative: Dr. Graf & Partner

(57) **Abstract**

An in-vivo sensing device that may insert error correction codes into, for example, data blocks containing, for example, image data. Data blocks may be transmitted from a body to an external receiving system or processing system that may decode the error correction codes, and may correct or account for data that may have been corrupted in the transmission.

## Description

### FIELD OF THE INVENTION

The present invention relates to a device, system and method of error detection and/or correction of transmitted data that may be collected in-vivo.

### BACKGROUND OF THE INVENTION

Transmission of data such as digital data over, for example, wireless media may result in the corruption of segments or portions of such data between the point of transmission of such data and the point of reception of such data. For example, the transmission of digitized video or image data over a wireless system may result in incomplete or otherwise corrupted bytes or other data units that make up the digitized video. Error correction coding techniques may detect and possibly correct errors that occur in such transmissions. In some encoding techniques, one or more symbols, bytes or other units of data may be added to a stream of data being transmitted. The value of the encoded data units may be used as a check on, for example, the content of bits or bytes of information that were transmitted, or on another aspect of the data being transmitted, A receiver may decode the encoded data and check the received data stream against the encoded values to ensure, for example, that the transmitted data was received correctly. In some error events detected errors may be corrected. Other methods or techniques of error detection and/or correction are possible.

### SUMMARY OF THE INVENTION

According to an embodiment of the invention a device, system and method is provided for including error correction coding techniques such as block coding or such as, for example, those based on Bose Chaudhuri Hocquenghem (BCH) coding schemes, in digitized data transmitted by an in-vivo sensing device such as, for example, an autonomous imaging capsule to an external receiver. Error correction codes may be included or encoded into the data that is produced and transmitted by, for example, an in-vivo sensing device. The encoded data may be received and decoded by, for example, a decoder associated with a receiver of the digitized data. The receiver may include a mechanism that may decode the transmitted data. Corrupted data may in some cases be partially or fully corrected, retransmitted or otherwise accounted for.

It should be appreciated that the term "error correction" or "error correcting" may be interpreted to include error detection and/or correction. The term "correction decoder" may be interpreted to include an "error detector".

### BRIEF DESCRIPTION OF THE DRAWINGS

The present invention will be understood and appreciated more fully from the following detailed description taken in conjunction with the drawings in which:
Fig. 1 shows a schematic diagram of an autonomous in-vivo sensing device including a transmitter and a receiver or receiver system that may collect data transmitted by an autonomous in-vivo sensing device, in accordance with an exemplary embodiment of the invention;
Fig. 2 is a schematic diagram of a block of data to which may include an error correction code algorithm in accordance with an exemplary embodiment of the invention;
Fig. 3 is a flow chart of a method of error conection of data transmitted by an in-vivo sensing device in accordance with an exemplary embodiment of the invention; and
Fig. 4 is a flow chart of a method of error detection according to an embodiment of the invention.

It will be appreciated that for simplicity and clarity of illustration, elements shown in the figures have not necessarily been drawn accurately or to scale. For example, the dimensions of some of the elements may be exaggerated relative to other elements for clarity, or several physical components may be included in one functional block or element Further, where considered appropriate, reference numerals may be repeated among the figures to indicate corresponding or analogous elements.

### DETAILED DESCRIPTION OF THE INVENTION

In the following description, various aspects of the present invention will be described. For purposes of explanation, specific configurations and details are set forth in order to provide a thorough understanding of the present invention. However, it will also be apparent to one skilled in the art that the present invention may be practiced without the specific details presented herein. Furthermore, well-known features may be omitted or simplified in order not to obscure the present invention. Images described in this application as video may also include still images or other image or sensory data.

It is noted that some embodiments of the present invention may be directed to an autonomous, typically swallowable in-vivo device. Other embodiments need not be swallowable. Devices or systems according to embodiments of the present invention may be similar to embodiments described in International Application WO 01/65995 and/or in U.S. Patent No. 5,604,531, each of which are assigned to the common assignee of the present invention and each of which are hereby fully incorporated by reference. Furthermore, a receiving and/or display system suitable for use with embodiments of the present invention may also be similar to embodiments described in WO 01/65995 and/or in U.S. Patent Number 5,604,531. Devices and systems as described herein may have other configurations and other sets of components.

Alternate embodiments of a device, system and method according to various embodiments of the invention may be used with other devices, non-imaging and/or non-in-vivo devices.

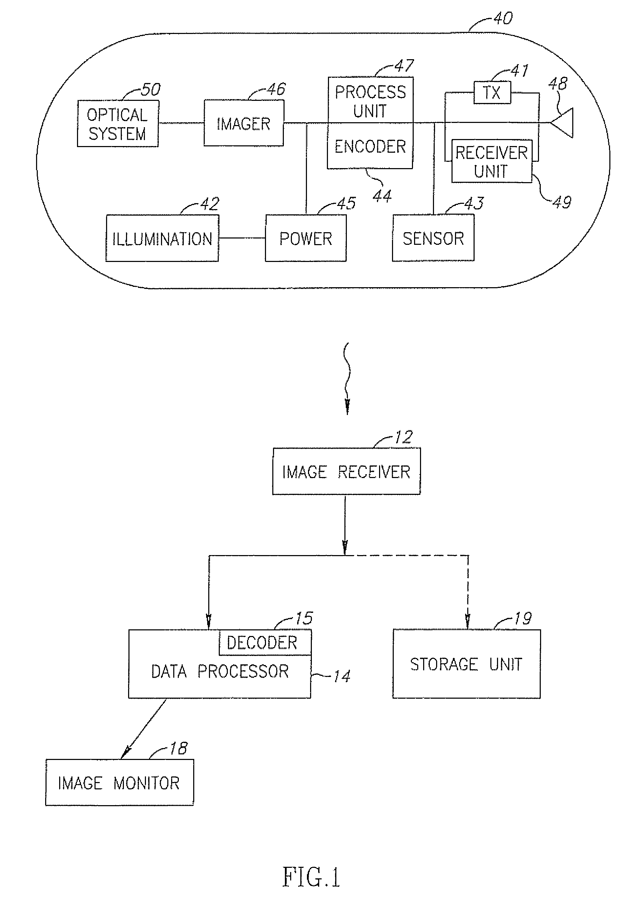

Reference is made to Fig. 1, which shows a schematic diagram of an embodiment of an in-vivo imaging device and a receiver system in accordance with an embodiment of the invention. In one embodiment, the system may include a device 40 having a sensor such as, for example, an imager 46, an illumination source 42, and a transmitter 41. In one embodiment the device 40 may include a receiver unit 49. In some embodiments, device 40 may be implemented using a swallowable capsule, but other sorts of devices or suitable implementations may be used. In some embodiments, other sensors 43 in addition to or instead of imager 46 such as, for example, pH, temperature, pressure or other physiological parameter sensors may be included in device 40.

Transmitter 41 may operate using radio waves, but in some embodiments, other wireless transmission media may be used.

Device 40 may include a processing unit 47 that may, for example, contain or process instructions. Such instructions may in some embodiments include algorithms such as, for example, compression algorithms or error correction code algorithms. In some embodiments the instructions embodying an error correction code may be included in an encoder 44 that may be part of or connected to processing unit 47. Encoder 44 may in some embodiments be a hardware component. In some embodiments, encoder 44 may be a software or combination hardware/softwate component. Processing unit 47 and encoder 44, or the functionality of these units, may be included in transmitter 41; in alternate embodiments such functionality may be placed in different units, for example processor 47 may be an integral part or included in imager 46. Transmitter 41 may also include control capability, for, for example controlling the various operations of device 40, although control capability may be included in a separate component or in other components, such as in the imager. In some embodiments the device 40 may be controlled through receiving unit 49, for example, as discussed below.

Device 40 typically is or includes an autonomous swallowable capsule, but may have other shapes, and need not be swallowable or autonomous. For example, device 40 may be a capsule or other unit where all the components are substantially contained within a container or shell, and where device 40 does not require a wired or cabled connection to, for example, receive power or transmit information. Device 40 may communicate with an external receiving and display system to provide display of data, control, or other functions. In one embodiment, device 40 may collect sensory data from the GI tract while the capsule passes through the GI lumen. Other lumens may be imaged. Other embodiments may have other configurations and capabilities. For example, components may be distributed over multiple sites or units. Control information may be received from an external source.

In an embodiment, imager 46 in device 40 may include or may be associated with an optical system 50 including for example a lens or set of lenses. Transmitter 41 may transmit images or other data to a receiver 12, which may send or transfer data to data processor 14 and/or to storage unit 19, which are, according to one embodiment, included in an external receiving unit or system. Transmitter 41 may include a suitable transmitter able to transmit images and/or other data (e.g., control data) to a receiving device. For example, transmitter 41 may include an ultra low power RF transmitter with high bandwidth input. Transmitter 41 may transmit via antenna 48.

According to some embodiments device 40 may be controlled by an external signal, which may be sent, for example through an external transmitter (not shown). An external signal can be received by receiver unit 49. According to some embodiments transmitter 41 may be a transceiver (for example, may include receiving unit 49). According to embodiments of the invention an external signal, which may be an RF signal or other suitable typically wireless transmission, may be used to activate and/or alter the operational mode of the in vivo device 40. Such activating, deactivating or altering operational modes may include for example, activating or deactivating one or more components of the in vivo device 40, increasing or decreasing transmission power, increasing or decreasing the power consumption, increasing or decreasing the level of illumination, increasing or decreasing the rate of sensing, such as, for example, increasing the data capture rate from, for example, 2 images per second to for example, 14 images per second, or altering the sensing parameters such as, for example, in the case of an in vivo image sensor, increasing or decreasing the illumination intensity of the light sources. Other operational modes may be changed and other data capture rates may be used.

Power source 45 may include one or more batteries. For example, power source 45 may include silver oxide batteries, lithium batteries, other suitable electrochemical cells having a high energy density, or the like. Other power sources may be used. For example, instead of internal power source 45 or in addition to it, an external power source may be used to transmit power to device 40.

Outside the patient's body may be a data receiver such as, for example, a receiver 12 (typically including or associated with an antenna or antenna array). Data processor 14 may analyze data received by receiver 12 and may be in communication with storage unit 19, transferring image data (which may be stored and transferred as for example frame data) or other data to and from storage unit 19. Data processor 14 may also provide the analyzed data to image monitor 18 where a user may view the images. Image monitor 18 may present an image such as, for example, video data of for example the GI lumen or other body lumen. In one embodiment, data processor 14 may be configured for real time processing and/or for post processing to be performed and/or viewed at a later time. Other monitoring and receiving systems may be used.

In some embodiments data processor 14 or another component may include, be connected to or be associated with a decoder 15. Decoder 15 or data processor 14 may include, be connected to or execute an algorithm capable of processing, for example, error correction codes. Decoder 15 or data processor 14 may be capable of evaluating transmitted data to determine whether data that was transmitted to it was corrupted. In some embodiments, decoder 15 may be included in or associated with receiver 12. Decoder 15 may be implemented in known methods, such as hardware (e.g., a processor, computer on a chip, etc.), software, firmware, or a combination of such elements. Decoder 15 may be included in another component, such as data processor 14 or may a separate component.

In some embodiments, there may be included in, for example, processing unit 47 or in another unit in device 40, instructions that when executed insert error detection and/or correction codes into data such as image or video data collected and transmitted by device 40. In some embodiments, error correction codes may be or include block codes such as for example those employing BCH coding schemes. Other suitable error correction codes or techniques may be used.

According to some embodiments an error detecting code may be included, for example in transmitter 41 or in another unit in device 40. An error detecting code, for example, the cyclic redundancy check (CRC) code, may be used to detect errors in transmitted data.

A system according to some embodiments of the invention includes an in-vivo sensing device transmitting information (e.g., images or other data) to a data receiver and/or recorder possibly close to or worn on a subject. A data receiver and/or recorder may of course take other suitable configurations. The data receiver and/or recorder may transfer the received information to a larger computing device, such as a workstation or personal computer, where the data may be further analyzed, stored, and/or displayed to a user. In other embodiments, each of the various components need not be required; for example, an internal device may transmit or otherwise transfer (e.g., by wire) information directly to a viewing or processing system.

Typically, device 40 transmits image information in discrete portions. Each portion typically corresponds to an image or frame. Other transmission methods are possible. For example, device 40 may capture an image once every half second, and, after capturing such an image, transmit the image to receiver 12 as an encoded image. Other constant and/or variable capture rates and/or transmission rates may be used. Typically, the image data recorded and transmitted is digital color image data, although in alternate embodiments other image formats (e.g., black and white image data) may be used. In one embodiment, each frame of image data includes 256 rows of 256 pixels each, each pixel including data for color and brightness, according to known methods. Other data formats may be used. For example, device 40 may transmit sensory data captured, for example, by sensor 43. Such data may include, for example, in vivo physiological data which may be transmitted as an electrical current, a voltage, etc.

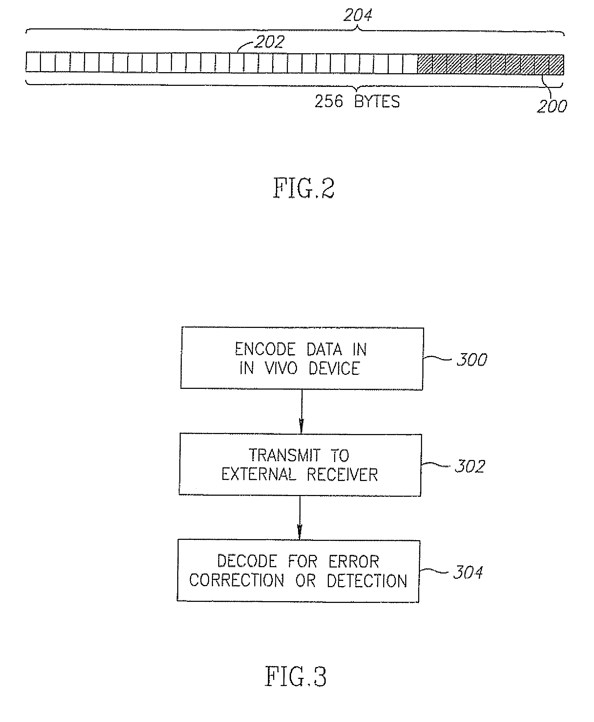

Reference is made to Fig. 2, a schematic diagram of a data block to which may have been added error correction data by an error detection and/or correction encoding algorithm in accordance with an exemplary embodiment of the invention. Error detection and/or correction data may include, for example, parity bits. In some embodiments, data such as, for example, sensory data or image data that may have been collected by a device 40 may be packaged or collected into blocks, for example, block 204. In some embodiments a block 204 of data may include 256 bytes of, for example, image data, which may be included in sub block 202. According to another embodiment block 204 may include 768 bytes of image data which may be included in sub block 202. Other block sizes or data formats may be used. A processor such as, for example, processing unit 47 or another component that may be included in device 40 may add to such block 204 of data an additional one or more bytes (or other data units) which may include error correction or detection data, such as parity bits. The bytes (or other data units) including error correction or detection codes may be included in sub block 200. In fig. 2 sub block 200 is located at the end of block 204 for illustrative purposes, however, according to embodiments of the invention, bytes including error correction or detection data may located in other locations within block 204. In some embodiments, approximately 1/5 of the bytes of block 204 may include error correction or detection codes. Other number of bytes or bits which include error correction or detection data may be used. In some embodiments, not all 8 bits in each byte of, for example, the bytes in sub block 202, may be protected. For example, in some embodiments only the most significant bits (e.g., 6 bits out of 8) may be protected by error correction coding.

Blocks 204 of data including sub block 200 which is added in accordance with error correction code algorithms may be transmitted, for example, by transmitter 41 to a receiver 12. The receiving unit may decode, reconstruct, correct or otherwise process the data, or such operations (or a portion of such operations) may be performed by a unit that receiver 12 passes the received data to, such as data processor 14.

According to some embodiments, original data may be modified by addition of an error correction or detection code, for example, by using extension coding. In some embodiments the original data, such as image data in a block or other data unit may be unmodified by the addition of error correction or detection codes, for example, by using systematic coding. In some embodiments, for example, when using systematic coding a receiver that is not equipped with error correction decoding algorithms or a decoder may receive and process the image data without interference from the data information symbols that may have been inserted by the error correction code algorithm. A component such as for example data processor 14 or another processor within or connected to receiver 12 may include instructions in for example a decoder 15, that when executed may decode data in a block 204. In some embodiments, processor 14 may be separate from receiver 12.

In some embodiments, error correction codes may be capable of correcting or accounting for up to 4 or 5 corrupted bits within block 204. Other numbers of corrected bits are possible.

Reference is made to Fig. 3, which is a flow chart of a method of including error correction codes in data transmitted by an in-vivo sensing device in accordance with an exemplary embodiment of the invention. In block 300, a component of an in-vivo sensing device, such as an encoder or a processing unit, may add data, such as, for example, parity bits or other error correction code symbols to, for example, a block of image data that was collected by the in-vivo device In some embodiments, the error correction codes may be based on or employ block coding, convolution coding, trellis coding, turbo coding, or another suitable coding scheme.

In block 302, the collected image data which includes error correction codes, (e.g., parity bits or other data inserted by the error correction code algorithm or an encoder) may be transmitted from the in-vivo imaging device to an external receiver.

In block 304, a data processor or other decoder that may be included or connected to a receiver may decode the received data.

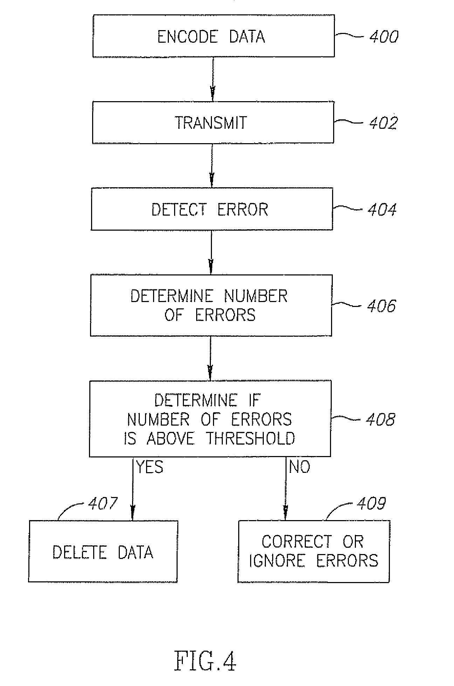

Reference is now made to Fig. 4, which is a flow chart of a method of error detection according to an embodiment of the invention. In block 400, a component of an in-vivo sensing device, such as an encoder or a processing unit, may add data, such as, for example, parity bits or other error correction and/or detection code symbols to, for example, a block of image data that was collected by the in-vivo device. The encoded data may be transmitted to an external receiving unit (block 402). A decoding algorithm and/or correcting code (e.g., CRC) may be applied to the transmitted data (block 404). In block 406 the amount (e.g., number or ratio) of errors is determined. The amount of errors may be determined to be above or below a predetermined threshold (block 408). The detection (406) and/or determination (408) may be carried out in decoder 15 or in any other appropriate component of the system according to embodiments of the invention. According to an embodiment of the invention, in block 407, a decision may be made, for example, in decoder 15, to delete or abort a row (e.g., sub block 202), a pixel (byte) or even an entire frame, if the amount of errors detected was determined to be above a predetermined threshold. According to other embodiments of the invention, if the amount of errors detected was determined to be above a predetermined threshold a decision made be made to perform a corrective action, e.g., by interpolating or employing other appropriate image processing or other signal processing techniques. For example, a decision may be made to substitute corrupted data, for example, by interpolation of correct data. Alternatively, in block 409 a decision may be made to ignore the errors detected if their amount was determined to be under a predetermined threshold. Alternatively, a decision can be made to correct the errors detected if their amount was determined to be under a predetermined threshold. For example, a predetermined threshold may be the maxirnal number that the specific error correction code can repair.

According to some embodiments the step of determining if the amount of errors is above or below a predetermined threshold (block 408) may be a trigger for activating and/or altering the operational mode of the in vivo device. For example, if the amount of errors detected is determined to be above a predetermined threshold a decision can be made to change transmission parameters, such as transmission power, transmission rate, the error correction algorithm to be used or other parameters for example, to improve the quality of transmission. According to embodiments of the invention decoder 15 or any other suitable component can , based on a determining step, send a signal, for example to receiver unit 49 (Fig. 1) to control parameters of the in vivo device operation (e.g., to alter transmission power, change transmission rate, etc.).

It will be appreciated by persons skilled in the art that the present invention is not limited to what has been particularly shown and described hereinabove. Rather the scope of the present invention is defined only by the claims, which follow:

## Claims

1. An autonomous in-vivo sensing device comprising
a sensor;
a transmitter; and
an error correction code encoder.

2. The device as in claim 1, wherein said encoder is to insert error detection codes into data transmitted by said device.

3. The device as in claim 2, comprising an imager and wherein said data includes image data.

4. The device as in claim 3, wherein said image data is unmodified by the error correction codes inserted in said data.

5. An in-vivo imaging system, comprising:
an autonomous in-vivo imaging device including an imager, a transmitter and an error correction encoder; and
a receiver unit to receive data from the imaging device, said receiver unit associated with an error correction decoder.

6. The system as in claim 5, comprising a processor to detect corrupted data.

7. A method for transmitting in vivo image data, the method comprising:
obtaining in vivo image data;
encoding said in vivo image data with an error detection code, thereby obtaining encoded image data; and
transmitting said encoded image data.

8. The method as in claim 7 wherein the transmitting is wireless.

9. The method as in claim 7 comprising decoding of transmitted images.

10. The method as in claim 9 wherein decoding transmitted images comprises detecting an amount of errors.

11. The method as is claim 7 comprising determining if an amount of errors is above a predetermined threshold.

12. The method as in claim 7 comprising deciding to ignore errors.

13. The method as in claim 7 comprising deciding to delete data.

14. The method as in claim 7 comprising deciding to substitute corrupted data.

15. The method as in claim 7 comprising deciding to change a transmission parameter.
